(19) Europäisches Patentamt
European Patent Office
Office européen des brevets

(11) Publication number: **0 010 358**
**A1**

# (12) EUROPEAN PATENT APPLICATION

(21) Application number: **79301944.9**

(22) Date of filing: **19.09.79**

(51) Int. Cl.³: **C 07 D 499/00**
A 61 K 31/43, C 07 D 205/08
C 07 D 498/04

(30) Priority: **20.09.78 GB 3750678**

(43) Date of publication of application:
**30.04.80 Bulletin 80/9**

(84) Designated Contracting States:
**AT BE CH DE FR GB IT NL SE**

(71) Applicant: **GLAXO GROUP LIMITED**
**Clarges House 6-12 Clarges Street**
**London W1Y 8DH(GB)**

(72) Inventor: **Watson, Nigel Stephen**
**5 Ruislip Road**
**Greenford Middlesex(GB)**

(74) Representative: **Holmes, Michael John et al,**
**Frank B. Dehn & Co. Imperial House 15-19 Kingsway**
**London WC2B 6UZ(GB)**

(54) **Beta-lactam compounds, processes for their preparation, compositions containing them, intermediates of use in their preparation and methods for the production thereof.**

(57) β-Lactam compounds, processes for their preparation, pharmaceutical compositions containing them and intermediates of use in their preparation are disclosed. More particularly, novel compounds of the general formula (I),

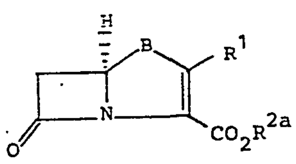

(I)

wherein $R^1$ represents a grouping of formula $-R^3.NH.R^4$ [where $R^3$ represents a group $-C_nH_{2n}-$ where n is an integer from 1 to 6 and $R^4$ represents an acyl group of formula $-CYR^a$ $-CY.ZR^b$ or $-CY.NHR^a$ (in which Y and Z, which may be the same or different, each represents oxygen or sulphur); $R^a$ represents a hydrogen atom; or an alkyl group which contains from 1 to 4 carbon atoms, optional substituents being selected from one or more of halogen, cyano, azido, amino, $C_{1-4}$ alkylthio, hydroxy, $C_{1-4}$ alkoxy and thiol; or an aralkyl group having up to 10 carbon atoms, optional substituents on the phenyl group when the aralkyl group is a benzyl group being selected from hydroxy of sulphonamido, optional substituents on the alkyl portion being selected from azido, amino, carboxyl or hydroxy; or an aryl group having up to 10 carbon atoms, optional substituents when the aryl group is a phenyl group being selected from halogen, cyano, $C_1$-4 alkoxy or $C_{1-4}$ alkyl; and $R^b$ represents a methyl or ethyl group]; $R^{2a}$ represents a carboxyl esterifying group or a hydrogen atom; and B represents a group S or S→O; and salts thereof are provided. The compounds have been found to be active against a range of gram-positive and gram-negative microorganisms and to be stable, in general, to the action of β-lactamases produced by some gram-positive and gram-negative organisms.

EP 0 010 358 A1

TITLE MODIFIED
see front page



"β-Lactam compounds, processes for their preparation, compositions containing them and intermediates of use in their preparation"

This invention relates to novel penems having antibiotic activity, to processes for their preparation, to pharmaceutical compositions containing them and to intermediates of use in the preparation of antibiotics.

According to one feature of the present invention there are provided compounds of general formula

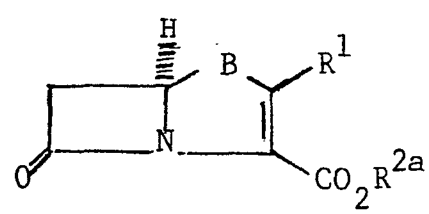

(I)

wherein

$R^1$ represents a grouping of formula $-R^3.NH.R^4$ [where $R^3$ represents a group $-C_nH_{2n}-$ where n is an integer from 1 to 6 and $R^4$ represents an acyl group of formula $-CYR^a$, $-CY.ZR^b$ or $CY.NH.R^a$ (in which Y and Z, which may be the same or different, each represents oxygen or sulphur); $R^a$ represents a hydrogen atom or

(a) an alkyl group which may contain from 1 to 4 carbon

atoms, for example, a methyl, ethyl, propyl or isopropyl, butyl, sec-butyl or tert-butyl group, optional substituents being for example selected from one or more of halogen e.g. fluorine, chlorine or bromine; cyano; azido; amino; hydroxy; $C_{1-4}$ alkoxy, thiol, and $C_{1-4}$ alkylthio,

(b) an aralkyl group having up to 10 carbon atoms especially an arylmethyl group e.g. a benzyl or substituted benzyl group, suitable substituents on the phenyl ring of a benzyl group being hydroxy, e.g. p-hydroxy, or sulphonamido, e.g. m-sulphonamido and suitable substituents on the methylene group of a benzyl group including azido, amino, carboxyl or hydroxy and protected forms thereof, or

(c) an aryl group having up to 10 carbon atoms e.g. a phenyl or substituted phenyl group, suitable substituents being halogen e.g. chlorine; cyano; alkoxy e.g. methoxy or alkyl e.g. methyl, and $R^b$ represents a methyl or ethyl group];

$R^{2a}$ represents a carboxyl esterifying group or a hydrogen atom; and

B represents a group $>S$ or $>S\rightarrow O$;

and salts thereof.

Compounds according to the invention may be of use as antibiotics as detailed below or may be useful as intermediates in the preparation of active compounds or in the purification of active compounds.

The compounds as shown in formula (I) have the 5R-configuration but the invention extends not only to such individual isomers but also to mixtures of

the compounds of formula (I) with the corresponding 5S-isomers, for example in racemic mixtures.

B preferably represents the group >S.

In the group $R^1$, the group $-C_nH_{2n}-$ represented by $R^3$ may have a straight or branched chain and n is preferably from 1 to 4 as, for example, in a methylene or ethylene group.

Groups $-NHR^4$ which are of particular interest include formamido, acetamido, ureido, methylureido and phenylureido, and formamido is especially preferred.

When $R^{2a}$ represents a carboxyl esterifying group, it may for example be an organic group derived from an alcohol (aliphatic or araliphatic), a phenol, a silanol or a stannanol. Such an alcohol, phenol, silanol or stannanol used to esterify the carboxyl group preferably contains not more than 24 carbon atoms.

Thus, the group $R^{2a}$ may represent a straight or branched unsubstituted or substituted alkyl or alkenyl group, preferably having from 1-8 carbon atoms, for example a methyl, ethyl, propyl, isopropyl, butyl, sec-butyl, t-butyl or allyl group, optional substituents being for example, alkoxy e.g. methoxy; halogen i.e. fluorine, chlorine, bromine or iodine; cyano; acyloxy, e.g. alkanoyloxy, such as acetoxy or pivaloyloxy, or alkoxycarbonyloxy, such as ethoxycarbonyloxy; acyl e.g. p-bromobenzoyl and alkoxycarbonyl e.g. ethoxycarbonyl;

an aralkyl group having up to 20 carbon atoms especially an arylmethyl group e.g. a benzyl or substituted benzyl group, suitable substituents being halo e.g. chloro; nitro, e.g. o or p-nitro; cyano; alkoxy e.g. p-methoxy or alkyl e.g. *p*-methyl groups; a diphenylmethyl or triphenylmethyl group or a fur-2-ylmethyl, thien-2-ylmethyl or pyrid-4-ylmethyl group, the heterocyclic groups of which may also be substituted e.g. by a $C_{1-4}$ alkyl group, preferably methyl;

an aryl group having up to 12 carbon atoms e.g. a phenyl or substituted phenyl group, suitable substituents being halo e.g. chloro; nitro, e.g. *o* or *p*-nitro; cyano; alkoxy e.g. *p*-methoxy or alkyl e.g. *p*-methyl groups;

a cycloalkyl group containing not more than 12 carbon atoms e.g. adamantyl, cyclohexyl or cyclopentyl;

a heterocyclic group containing not more than 12 carbon atoms, the heteroatom being, for example, oxygen, as in the tetrahydropyranyl or phthalidyl group;

a stannyl group having up to 24 carbon atoms for example a stannyl group carrying three substituents which may be the same or different selected from alkyl, alkenyl, aryl, aralkyl, cycloalkyl, alkoxy, or aralkoxy groups. Such groups include methyl, ethyl, propyl, n-butyl, phenyl and benzyl groups;

or a silyl group having up to 24 carbon atoms which may carry three groups which may be the same or different selected from alkyl, alkenyl, cycloalkyl, aralkyl and aryl groups. Such groups will preferably be $C_{1-4}$ alkyl e.g. methyl, ethyl, n-propyl, iso-propyl or *t*-butyl groups.

The alkyl, alkoxy, alkanoyloxy, alkanoyl and alkoxycarbonyl substituents referred to above preferably contain 1 to 4 carbon atoms.

Where substituents are present on any of the above-mentioned groups, there will preferably be only one of each of the substituents, with the exception of halogen, in which case there may be up to three substituents.

Salts of compounds of formula (I) include, for example, salts of acidic compounds of the invention formed with inorganic and organic bases. Such salts include alkali metal salts, e.g. sodium, potassium and lithium salts, alkaline earth metal salts, e.g. calcium and magnesium salts, ammonium salts, and substituted ammonium salts. Salts of compounds of formula (I) also include acid addition salts of compounds of formula (I) in which the $R^1$ group contains a basic nitrogen atom. Examples of acid addition salts are those formed with hydrochloric, hydrobromic, sulphuric, phosphoric, lactic, citric, fumaric, maleic, p-toluenesulphonic and trifluoroacetic acids. Compounds of formula (I) in which $R^{2a}$ is hydrogen and $R^1$ carries a basic nitrogen atom may exist in zwitterionic forms and such zwitterionic forms are included within the present invention.

In general acids and metabolically labile esters of compounds of formula (I) in which B represents $>S$, and salts of such compounds are the preferred forms for use in medicine. However, other compounds of the invention are also useful, particularly for the preparation and purification of the preferred compounds just referred to and other compounds. Thus for example, where the ester grouping is readily cleaved e.g. by hydrolysis or hydrogenolysis, without significant degradation of the

rest of the molecule, the esters are especially useful as carboxyl-protected derivatives of the parent compounds. Those esters which are readily cleaved and are primarily of use in this connection include arylmethyl esters, especially the benzyl, p-nitrobenzyl, benzhydryl and trityl esters as well as the stannyl, e.g. tri-n-butylstannyl, and silyl esters. As indicated above, the ester grouping may be metabolically labile, i.e. it may be converted into the carboxylic acid during general metabolic processes e.g. in the blood or liver. Metabolically labile esters include substituted alkyl esters carrying an oxygen substituent on the α-carbon atom, for example α-acyloxyalkyl, e.g. acetoxymethyl and pivaloyloxymethyl esters; α-alkoxycarbonyloxyalkyl esters, e.g. 1-ethoxycarbonyloxyethyl esters; and phthalidyl esters.

Compounds of formula (I) wherein B represents >S and $R^{2a}$ represents a hydrogen atom or a metabolically labile ester grouping, together with salts of such compounds, in general possess antibacterial activity. They have been found to be active against a range of gram-positive and gram-negative microorganisms, for example against strains of Staphylococcus aureus, Micrococcus species, Escherichia coli, Salmonella typhimurium, Shigella sonnei, Enterobacter cloacae, Klebsiella aerogenes, Prteus mirabilis, Proteus vulgaris, Serratia marcescens, Probidence species and Haemophilus influenzae.

In general, the compounds are stable to the action of β-lactamases produced by some gram-positive organisms

for example those produced by Staphylococcus aureus, and to β-lactamases produced by some gram-negative organisms such as Enterobacter cloacae.

Compounds of formula (I) wherein B represents >S and $R^{2a}$ represents a hydrogen atom or a metabolically labile ester grouping and physiologically acceptable salts of such compounds may thus be formulated as pharmaceutical (including veterinary) compositions which may if desired contain a further antibiotic. Such compositions will also contain carriers and/or excipients and may, for example, be in a form adapted for oral or parenteral administration.

Such compositions may thus, for example, take the form of powders, tablets, capsules, lozenges, solutions and syrups suitable for oral administration, and may include, for example, starch, lactose, talc, magnesium stearate, gelatin, distilled water and suspending, dispersing, emulsifying, flavouring or colouring agents.

Active compounds of formula (I) may further be formulated in rectal compositions such as suppositories or retention enemas.

For parenteral administration, the compounds may be presented in ampoules for formulation before use.

The active compounds of formula (I) will generally be administered to humans at a total daily dosage level of 50 mg to 20 g, preferably from 100 mg to 10 g, which may be in divided doses given 1-4 times per day. Dosage units will in general contain 12.5 mg to 5 g, preferably

50 mg to 1 g of active compound according to the invention.

Active compounds of formula (I) may be of use in treating a variety of diseases, in humans and animals, caused by pathogenic bacteria, such as respiratory tract or urinary tract infections.

The compounds of the invention may be prepared by two principal methods, namely by a ring-closure and by acylation of corresponding penems carrying a free amino group. In general they may be obtained from available starting materials, as illustrated in the reaction scheme hereinafter

The compounds of general formula (I) wherein B represents a group >S may thus, for example, be prepared by reacting a compound of the formula (II)

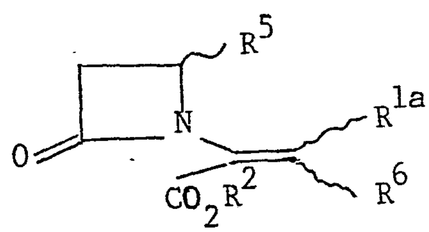

(II)

[wherein

$R^{1a}$ is as defined above for $R^1$ (except that any amino, carboxyl, hydroxy or thiol groups should be in protected form) or represents a group of formula $-R^3.R^c$ (where $R^3$ is as defined above and $R^c$ represents an azido or protected amino group);

$R^2$ represents a carboxyl esterifying group as defined above for $R^{2a}$;

$R^5$ represents a halogen atom or a group of formula $-\overset{\oplus}{N}R^xR^yR^z$ (in which $R^x$, $R^y$ and $R^z$, which may be the same or different, each represents an aliphatic, araliphatic

or aromatic group or two of $R^x$, $R^y$ and $R^z$ together with the nitrogen atom to which they are attached represent a five-, six- or seven-membered heterocyclic ring optionally containing a further heteroatom and the third of $R^x$, $R^y$ and $R^z$ is as defined above or $R^x$, $R^y$ and $R^z$ together with the nitrogen atom to which they are attached form an aromatic heterocyclic ring or an aromatic or non-aromatic polycyclic heterocyclic system);

and $R^6$ represents a leaving group, for example a halogen atom or an acylated or etherified hydroxy group] with hydrogen sulphide (where necessary in the presence of a base) or with a hydrosulphide or sulphide salt, followed where $R^{1a}$ is different from $R^1$ in the desired compound of formula (I), by conversion to a group of formula $-R^3NH_2$ (where $R^3$ is as hereinbefore defined) and subsequent acylation to give the desired compound of formula (I), deesterification being optionally effected before, during or after said conversion of a group $R^{1a}$ into a group $-R^3NH_2$ or $R^1$.

Where a compound of formula (II) is employed which is racemic, the product of the invention obtained will likewise be racemic.

If desired, a compound of formula (I) in which B represents $>S$ obtained by the above reaction may subsequently be oxidised, as discussed in more detail hereinafter, to yield a compound of formula (I) in which B represents a group $>S\rightarrow O$. Other optional subsequent reaction steps include; reaction of a free acid of formula (I) with a base or reaction of a compound of formula (I) wherein the $R^1$ group contains a basic nitrogen atom with an acid to yield a salt; and/or

modification of the $R^1$ group, including deprotection of a compound in which $R^1$ carries one or more protected carboxyl, amino, hydroxy or thiol groups to yield a compound in which $R^1$ carries one or more free carboxyl, amino, hydroxy or thiol groups; and/or separation of a mixture of 5R- and 5S-isomers to yield an individual 5R-isomer or formula (I); all of which optional steps are discussed in more detail hereinafter.

Suitable hydrosulphide and sulphide salts include, for example, alkali metal, e.g. sodium, potassium and lithium salts; alkaline earth metal, e.g. calcium, salts; and onium salts, for example quaternary ammonium salts wherein the quaternary ammonium ion has the formula

R' ⊕
|
R"-N-R where R, R', R" and R"', which may be the
|
R"'

same or different, each represents an alkyl or aralkyl group. Examples of such salts include the benzyltrimethylammonium and tetra-n-butylammonium salts.

In the reaction with hydrogen sulphide, the presence of a base, either organic or inorganic, is desirable to accelerate the reaction, to optimise yields and to curtail excessive acidity. It is in general preferred to use as weak a base as possible which is effective in the reaction. Suitable bases for use in the reaction include amine bases, for example, tertiary amine bases, e.g. trialkylamines, preferably those wherein the alkyl groups have from 1 to 4 carbon atoms such as triethylamine, hindered bases such as 3-azabicyclo-[3,2,2]-nonane or aromatic bases such as pyridine.

Where $R^5$ represents a halogen atom, the reaction will in general be effected at a temperature of from -150°C to +50°C, reaction temperatures of from -50°C to +10°C, particularly -20°C to +10°C, being preferred.

The reaction is desirably effected in the presence of an inert solvent, stable to the reaction conditions employed. Suitable solvents include, for example, cyclic ethers e.g. tetrahydrofuran and dioxan, halogenated hydrocarbons, e.g. methylene chloride and 1,2-dichloroethane, esters, e.g. ethyl acetate and amide solvents, e.g. dimethylformamide. Alternatively, where the reaction is effected in the presence of a base, an excess of base may serve as solvent.

Where $R^5$ represents a group of formula $-\overset{\oplus}{N}R^xR^yR^z$, the reaction with hydrogen sulphide or a hydrosulphide or sulphide salt is preferably carried out at temperatures of from -30° to +30°C. The reaction is completed by cyclisation to form a penem of formula (I) by heating. The cyclisation reaction is preferably effected above +40°C, e.g. at +50° to +100°C.

Following the reaction of the compound of formula (II) wherein $R^5$ represents a group of formula $-\overset{\oplus}{N}R^xR^yR^z$ with the hydrogen sulphide or hydrosulphide or sulphide salt, it may be possible to isolate a thioenolate of the formula (IIA)

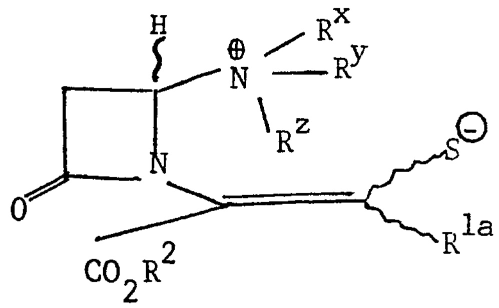

(IIA)

(wherein $R^x$, $R^y$, $R^z$, $R^{1a}$ and $R^2$ have the above meanings). On heating, e.g. in a solvent such as ethyl acetate or 1,2-dichloroethane, the compound of formula (IIA) cyclises to the desired penem ester of formula (I).

Cyclisation may if desired be carried out in the presence of an acid scavenging agent, e.g. an insoluble inorganic base such as solid, anhydrous sodium, potassium or calcium carbonate.

In the compounds of formula (II) and (IIA), when $R^{1a}$ represents a group of formula $-R^3R^c$ wherein $R^c$ represents a protected amino group, the latter will be cleavable to $-NH_2$, for example by hydrolysis or reduction, before or after cyclisation. Many acyl groups $R^4$ will be similarly removable and may, indeed be removed, for example after cyclisation, to permit introduction of a more preferred acyl group $R^4$.

When $R^5$ represents a halogen atom it is preferably a chlorine or bromine atom.

In the case that $R^5$ represents a group of formula $-\overset{\oplus}{N} R^xR^yR^z$ there will be an anion $A^\ominus$ associated with the compound of formula (II). In general the anion $A^\ominus$ will be derived from the reagent used for the introduction of the group $R^6$ e.g. a halide ion such as a chloride ion, as described hereinafter.

Where $R^5$ represents a group of formula $-\overset{\oplus}{N} R^xR^yR^z$, $R^x$, $R^y$ and $R^z$ may, for example each represent an alkyl group having up to 8 carbon atoms. e.g. a $C_{1-6}$ alkyl group preferably a $C_{1-4}$ group, an aralkyl group having up to 6 carbon atoms in the alkyl portion or an aryl group, such aralkyl and aryl groups desirably being monocyclic, e.g. benzyl and phenyl, and such alkyl groups include also

cycloaliphatic e.g. $C_{3-7}$ cycloalkyl groups; or two of $R^x$, $R^y$ and $R^z$ may together with the nitrogen atom to which they are attached represent a five, six- or seven-membered heterocyclic ring optionally containing a further oxygen or sulphur atom e.g. a N-alkyl-piperidinium or N-alkyl-morpholinium group; or $R^x$ $R^y$ and $R^z$ may, together with the nitrogen atom to which they are attached represent a bicylic, non-aromatic heterocyclic ring system e.g. a quinuclidinium group, or an aromatic ring e.g. pyridinium. A triethylammonium group is particularly preferred.

$R^6$ is preferably the residue of an acid $R^6H$ having a $pK_a$ (in water at 25°C) of 3.5 or less.

Where $R^6$ represents an acylated or etherified hydroxy group this may, for example, be an aliphatic, cycloaliphatic, aromatic or araliphatic carbonyloxy, sulphonyloxy or phosphinyloxy group containing 1 to 20 carbon atoms or an aliphatic ether group containing 1 to 6 carbon atoms. e.g. methoxy. Suitable sulphonyloxy groups include, for example, those wherein the aliphatic or aromatic grouping is an alkyl (e.g. $C_{1-8}$) group, which may be substituted by a halogen atom e.g. fluorine or chlorine, or an aryl (e.g. $C_{6-15}$) group which may carry alkyl, e.g. methyl, alkoxy e.g. methoxy or halogen e.g. bromine substituents. Suitable phosphinyloxy groups include, for example, groups of formula $-O\overset{\overset{O}{\|}}{P}(R^8)-R^7$ where $R^7$ and $R^8$, which may be the same or different, may each represent an alkyl, aryl or aralkyl group or may together with the phosphorus atom form a 5- or 6-membered ring.

Alternatively $R^7$ and/or $R^8$ may represent groups $OR^e$ and $OR^f$ respectively where $R^e$ and $R^f$ are as defined for $R^7$ and $R^8$ or may together form a divalent 5- or 6-membered ring. An example of the latter type of phosphinyloxy group is the group

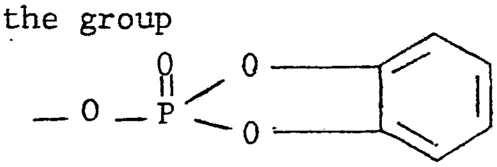

Suitable carbonyloxy groups include, for example, those wherein the aliphatic or aromatic grouping is an alkyl (e.g. $C_{1-8}$) group optionally substituted by one or more halogen atoms or an aryl (e.g. $C_{6-15}$) group optionally substituted by, for example, one or more halogen atoms or nitro groups.

When $R^6$ represents a halogen atom, this is preferably a chlorine or bromine atom.

Preferred meanings for $R^6$ include methanesulphonyloxy, trifluoromethanesulphonyloxy, *p*-toluenesulphonyloxy, benzenesulphonyloxy, dimethoxyphosphinyloxy groups and a chlorine atom.

The products of the ring-closure reaction will be esters. Where a free acid of formula (I) or a salt of such an acid is ultimately required, the ester may subsequently be cleaved at any stage after the ring-closure. Where an ester of a compound of formula (I) is required different from that originally produced, this may be obtained by deesterification followed by reesterification as described hereinbelow

Suitable methods for deesterification are well known in the art. Thus, for example, a silyl or stannyl ester may be cleaved by mild solvolysis e.g. by reaction with water, alcohols, phenols or carboxylic acids e.g. acetic

acid. Alternatively, esters which are readily subject to reduction, for example, arylmethyl esters such as benzyl, benzhydryl, trityl or more preferably, p-nitrobenzyl esters may be cleaved by reduction e.g. by hydrogenolysis, for example using a metal catalyst, e.g. a noble metal such as platinum, palladium or rhodium. The catalyst may be supported e.g. on charcoal or kieselguhr.

In the case of p-nitrobenzyl esters, cleavage may also be effected by reduction of the nitro group (e.g. using a dissolving metal reducing agent such as zinc in acetic acid or zinc in aqueous tetrahydrofuran or acetone controlled in the pH range 3-6, preferably 4-5.5 e.g. by the addition of aqueous HCl; aluminium amalgam in a moist ether, e.g. tetrahydrofuran; or iron and ammonium chloride in an aqueous ether such as aqueous tetrahydrofuran) followed by hydrolysis either under the reduction conditions or by subsequent treatment with acid. Alternatively, a p-nitrobenzyl ester may be cleaved under alkaline conditions using, for example, sodium sulphide in a solvent such as aqueous tetrahydrofuran, dimethylformamide or acetone.

As indicated hereinafter, reductive cleavage of an ester may result in simultaneous reduction of an azido group or reductive cleavage of a protected amino group in $R^1$. Similarly hydrolysis may simultaneously cleave a protected amino group in $R^1$ or the group $-NHR^4$ itself and care must therefore be taken to cleave the ester selectively where such simultaneous cleavage is not desired.

Where it is desired to produce a salt of the compound of formula (I), an acid may be reacted, e.g. in an

appropriate organic solvent with an appropriate base, preferably in equimolar quantities and preferably under conditions favouring precipitation of the salt. In the formation of alkali metal salts for example, e.g. sodium or potassium salts, an alkanoate is a preferred base e.g. a 2-ethyl hexanoate.

Where the compound of formula (I) obtained is a compound in which the $R^1$ group contains a basic nitrogen atom, it may, if desired, subsequently be converted into an acid addition salt by reaction with an appropriate acid.

Sulphoxides of formula (I) according to the invention may be prepared from the corresponding sulphides of formula (I) by, for example, oxidation with peracids such as peracetic, monoperphthalic, m-chloroperbenzoic or metaperiodic acid. Oxidation is preferably effected at a temperature not greater than 10°C and avoiding excess oxidising agent.

Esters of the acid of formula (I) may, for example, be prepared by reaction with an alcohol, phenol, silanol or stannanol or a reactive derivative thereof to form the desired ester. Reaction will desirably be effected under mild conditions in order to prevent rupture of the bicyclic nucleus. The use of neutral or mild acidic or basic conditions therefore, at temperatures between -70° and +35°C is preferred.

The alkyl, alkoxyalkyl and aralkyl esters may be prepared by reaction of the acid of formula (I) with the appropriate diazoalkane or diazoaralkane e.g. diazomethane or diphenyldiazomethane. The reaction will generally be effected in an ether, ester or a halohydrocarbon solvent,

e.g. diethyl ether, ethyl acetate or dichloromethane. In general, reduced temperatures are preferred, for example, -15° to +15°C.

The esters derived from alcohols may also be produced by reaction of a reactive derivative of the alcohol, for example, a halide such as the chloride, bromide or iodide or a hydrocarbonsulphonyl derivative such as a methanesulphonyl or p-toluenesulphonyl ester, with a salt of the acid of formula (I) e.g. an alkali or alkaline earth metal salt such as a lithium, sodium, potassium, calcium or barium salt or an amine salt, such as a triethylammonium salt. This reaction is preferably carried out in a substituted sulphoxide or amide solvent, e.g. dimethyl sulphoxide, dimethylformamide or hexamethylphosphoramide. Alternatively, the esters may be prepared by reaction of the acid with the alcohol in the presence of a condensing agent such as dicyclohexylcarbodiimide.

Stannyl esters may conveniently be formed by reaction of the carboxylic acid of formula (I) or a salt thereof with reactive tetravalent tin moieties. Trialkyl tin oxides are preferred for the synthesis of tin compounds in view of their availability and low toxicity.

Silyl esters of the acid of formula (I) may be formed by reaction with a reactive tetravalent silicon moiety. Trialkylsilyl halides and mono- or bis-silylated acetamides are preferred as silylating agents. Proton acceptors e.g. weak bases such as pyridine may often be used with advantage when hydrogen halides are formed during such esterification.

Where the initial product of the ring-closure is a

compound corresponding to formula (I) but wherein $R^1$ represents a group of formula $-R^3R^c$, such a compound may be further reacted in order to convert the group of formula $-R^3R^c$ into a group of formula $-R^3NH_2$ which group may then be acylated to produce the desired compound of formula (I).

Separation of a mixture of a 5R-isomer of formula (I) with the corresponding 5S-isomer may be effected, for example, by known techniques such as fractional crystallisation of a salt of an acid of the invention formed with an optically active base. Methods of separation and resolution are described, for example, in "Tables of Resolving Agents and Optical Resolutions" by S. H.Wilen, published by University of Notre-Dame Press 1972.

Acylation of a compound of formula (IA)

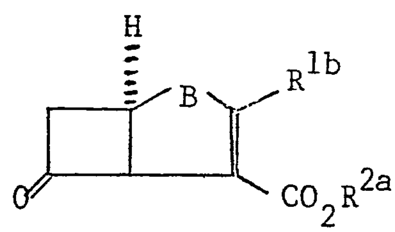

(IA)

wherein $R^{1b}$ is a grouping of formula $-R^3NH_2$, where $R^3$ has the above meaning, and $R^{2a}$ and B have the above meanings, constitutes a further feature of the invention. The 5R-isomer of formula (IA) may be reacted alone or in admixture with the corresponding 5S-isomer. Where a mixture of isomers is acylated but only the 5R-isomer of formula (I) is required, separation may be effected, for example as described above. Where $R^{2a}$ is a hydrogen atom, the compound of formula (IA) exists as a zwitterion.

Acylation may be effected by reaction with an acylating agent e.g. of formula $X\text{-}C{=}Y.R^a$, $X.CY.Z\ R^b$ or

X-CYNHR$^a$ (where Y, Z, $R^a$ and $R^b$ are as hereinbefore defined and X represents a displaceable substituent, for example, a halogen atom, e.g. chlorine or bromine, an O-acyl group, an O-hydrocarbyl group or a hydroxy group. Thus for example, the acylating agent may be a carboxylic or thiocarboxylic acyl halide, carbamoyl halide, thiocarbamoyl halide, or haloformyl ester (X = halogen); a carboxylic acyl anhydride, which may be symmetrical or mixed (X = O.Acyl); a carboxylic or thiocarboxylic acid in the presence of a coupling agent such as dicyclohexylcarbodiimide (X = OH); or an active ester of a carboxylic or thiocarboxylic acid, such as 4-nitrophenylformate. The acylating agent may, however, also be a ketene or sulphene or an isocyanate or isothiocyanate or an activated imide.

In order to avoid complicating side-reactions where it is desired to prepare a compound of formula (I) in which $R^{2a}$ represents a hydrogen atom, the acylation reaction is often more conveniently carried out before removal of the carboxyl esterifying group. The use of compounds of formula (IA) having an ester group $COOR^2$ is particularly preferred in reactions involving the use of a carboxylic or thiocarboxylic acid in the presence of a coupling agent such as dicyclohexylcarbodiimide; an arylmethyl ester group, e.g. a benzyl ester group, is preferred. Acylation with an acyl anhydride or an isocyanate may, however, readily be effected on the free acid in which $R^{2a}$ is hydrogen.

In many cases, acylation may be assisted by the presence of a bis-trialkyl tin oxide, such as bis-

BAD ORIGINAL

tri-*n*-butyl tin oxide.

Activated imides will generally be N-acyl cyclic imides. Such compounds may have the formula $R^dR^eN.CY.R^f$, wherein $R^d$ and $R^e$ together with the nitrogen atom to which they are attached, form a heterocyclic ring, in which the nitrogen is linked to two carbonyl groups, Y represents oxygen or sulphur and $R^f$ represents $R^a$ or $ZR^b$ where $R^a$, $R^b$ and Z are as defined above.

In some cases it may be preferable to form an activated derivative of the parent amine prior to acylation, e.g. a $C_{1-5}$ trialkylsilyl derivative such as a trimethylsilyl derivative. Carboxylic acyl, thiocarboxylic acyl, or carbamoyl halide reagents are preferably the chlorides. Mixed anhydride reagents preferably contain the acyl group to be introduced attached to a tertiary-alkanoyloxy or $C_{1-5}$ alkoxycarbonyloxy grouping, e.g. pivaloyloxy or ethoxycarbonyloxy. Reactive ester reagents include aryl esters, especially aryl esters carrying electron withdrawing substituents e.g. nitrophenyl esters. Reactive ester reagents prepared from N-hydroxyimides e.g. N-hydroxysuccinimide and from other reagents e.g. 1-hydroxybenzotriazole or 2-mercaptopyridine, may also be used. The reactive ester reagent may conveniently be formed *in situ.*

It may be convenient to carry out the reaction in the presence of a base, preferably a weak base, e.g. an inorganic base such as an alkali metal carbonate or bicarbonate or an organic base e.g. a tertiary base such as a trialkylamine, particularly when the aminoacid (zwitterion) form of the parent amine or an acid addition

BAD ORIGINAL

salt thereof is used as starting material or, where the acylating agent liberates acid, the base thereby functioning as an acid binding agent.

Suitable solvents for the reaction include aprotic solvents such as an amide, e.g. dimethylformamide, dimethylacetamide or hexamethylphosphoramide; an ester, e.g. ethyl acetate; an ether, e.g. tetrahydrofuran or dioxan; a ketone, e.g. acetone; a halogenated hydrocarbon, e.g. dichloromethane or chloroform; a substituted sulphoxide, e.g. dimethylsulphoxide; or mixtures of the above solvents. Reactions with acyl halides and anhydrides may also be effected in aqueous solutions, e.g. aqueous acetone or tetrahydrofuran. The reaction is advantageously effected at or below room temperature e.g. in the range -20° to +35°C.

When, in a compound corresponding to formula (I) but in which $R^1$ represents a group $R^3R^c$, the group $R^c$ represents an azido group, conversion of the azido group into an amino group is effected by means of reduction, for example by catalytic hydrogenation. The hydrogenation catalyst is normally a noble metal catalyst, e.g. palladium, platinum or rhodium, or another transition metal catalyst such as nickel. The catalyst may be supported e.g. on charcoal or kieselguhr; the metal catalyst is preferably palladium e.g. as 10% palladium on charcoal. Hydrogenation will desirably be effected in a solvent for the starting material.

Suitable solvents for the hydrogenation include ethers such as diethyl ether or tetrahydrofuran; esters such as ethyl acetate; ketones such as acetone or methyl

BAD ORIGINAL

ethyl ketone; chlorinated hydrocarbons such as methylene chloride; amides such as dimethylformamide or dimethylacetamide; or alcohols such as ethanol; or mixtures thereof. Such solvents may advantageously be mixed with water or an aqueous buffer. In some combinations a two phase system may result. In cases where the substrate is sufficiently water soluble, water or an aqueous buffer may be used alone.

The azido group may alternatively be reduced using dissolving metal reducing agents e.g. zinc and aqueous hydrochloric acid controlled in the pH range 2 to 6, preferably 4-4.5. Suitable solvents include, for example, water-miscible solvents such as ketones e.g. acetone, ethers such as tetrahydrofuran and alcohols such as ethanol.

Certain esters used as starting materials may be cleaved during reduction, e.g. arylmethyl esters such as p-nitrobenzyl esters, to yield a free carboxyl group. Selective reduction of the azide group is possible where the ester group is not cleavable by reduction or only cleavable under different conditions from those used to reduce the azide group.

The acid product of formula (IA) may be isolated from solution by, for example, partitioning between the solvent (where water immiscible) and water and after removal of catalyst, the aqueous phase may be lyophilised to yield the desired acid.

The acid product of formula (IA) may also be isolated from solution by precipitation or crystallisation either as its zwitterionic form or as a salt as defined

BAD ORIGINAL

above. Depending on conditions the isolated compound may be in the form of a solvate or hydrate.

Where, in a compound corresponding to formula (I) but in which $R^1$ represents a group $R^3R^d$, the group $R^d$ represents a protected amino group, deprotection may be effected by conventional methods. Thus, for example a benzyloxycarbonyl group may be removed by catalytic hydrogenation, while the t-butoxycarbonyl group may be removed by acid hydrolysis. Such deprotection may, if desired, be accompanied by simultaneous cleavage of the ester group $-COOR^{2a}$ or selective deprotection may be effected.

Where a compound of formula (I) is formed in which $R^1$ carries a carboxyl, amino, hydroxy or thiol group in protected form and such a group is required in the free form, deprotection may subsequently be effected, for example using conventional techniques such as hydrolysis or hydrogenolysis. Thus, for example, deesterification and deprotection of an amino group may be effected by the methods described above in relation to $R^{2a}$ and $R^d$ respectively.

The compounds of general formula (II) (used as starting materials) may be prepared by a variety of methods. Thus they may, for example, be prepared by reaction sequences involving clavulanic acid and its derivatives or they may be prepared by total synthesis.

One method of preparing compounds of general formula (II) wherein $R^5$ represents a halogen atom involves reacting a compound of formula (III)

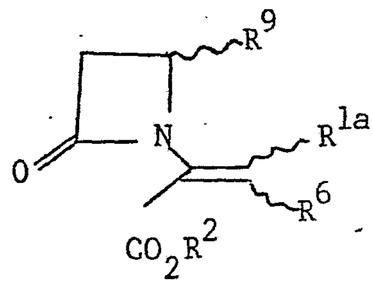

(III)

(wherein $R^{1a}$, $R^2$ and $R^6$ are as hereinbefore defined and $R^9$ is a group of formula $-SR^{10}$ or

$-\overset{O}{\overset{\uparrow}{S}}-R^{10}$ where $R^{10}$ represents an aliphatic, araliphatic, aromatic or heterocyclic group) with an electrophilic halogenating agent.

The halogenating agent may, for example, be molecular halogen or an N-halo amide or imide. The N-halo amide or N-halo imide may include a cyclic system, the amide or imide linkage forming part of the cyclic system; examples of such N-halo amides include N-bromocaprolactam and examples of such N-halo imides include the 1,3-dibromo-5,5-di-(lower alkyl) hydantoins (e.g. 1,3-dibromo-5,5-dimethyl-hydantoin); N-bromosuccinimide; N-chlorosuccinimide; N-bromophthalimide etc. Other useful N-halo amides include N-halo lower alkanoamides e.g. N-bromoacetamide.

The reaction will preferably be carried out in an inert solvent, e.g. a halogenated hydrocarbon, for example, methylene chloride.

Molecular halogen, e.g. chlorine or bromine, may, for convenience, be added in solution, e.g. in a halogenated hydrocarbon, such as carbon tetrachloride.

Suitable reaction temperatures are from -150°C to 25°C. The use of low temperature e.g. below about 0°C, has been found advantageous in assisting the control of

the reaction.

Where molecular halogen is used, it may be desirable to carry out the reaction in the presence of an acid binding agent in order to remove any hydrogen halide as it is formed and thus prevent side reactions. Suitable acid binding agents include amides e.g. carboxvlic acid amides such as acetamide, metal carbonates e.g. calcium carbonate, oxiranes e.g propylene oxide, and molecular sieves.

The acid binding agent, when used, will desirably be present in a quantity which ensures as rapid removal of hydrogen halide as possible.

When $R^{10}$ represents an aliphatic group, this may be saturated or unsaturated and thus may, for example, be an alkyl or alkenyl group which may contain 1-6 carbon atoms e.g. $C_{1-4}$ alkyl such as methyl or butyl, or a cycloalkyl group, which may have 3-7, preferably 5 or 6 carbon atoms. Such alkyl groups may, optionally, carry one or more etherified hydroxy groups.

When $R^{10}$ represents an araliphatic or aromatic group, it will desirably be an aralkyl group which may have 1-6 carbon atoms in the alkyl portion, or an aryl group, the rings in such aryl and aralkyl groups, preferably being monocyclic, e.g. phenyl; and optionally carrying substituents such as $C_{1-4}$ alkyl groups e.g. methyl groups.

When $R^{10}$ represents a heterocyclic group, it will desirably contain a carbon-attached 5-7 membered heterocyclic ring containing one or more heteroatoms such as nitrogen, sulphur or oxygen, which may be aromatic e.g. pyridyl and/or carry one or more alkyl groups, for example

BAD ORIGINAL

containing 1-6 carbon atoms e.g. a methyl group or may have a bicyclic structure, an example of such a group being a benzothiazol-2-yl group.

Specific groups $R^9$ which may be mentioned include, for example, methylsulphinyl; p-tolylthio; $C_{1-4}$ alkylthio e.g. n-butylthio, methylthio and ethylthio and benzylthio groups.

Compounds of formula (III) may, for example, be prepared from compounds of formula (IV),

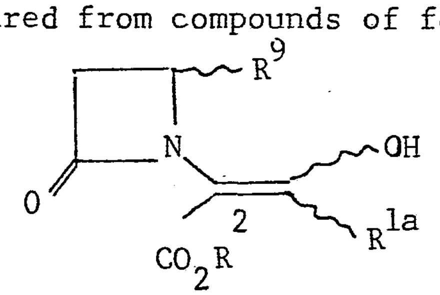

(IV)

(wherein $R^{1a}$, $R^2$ and $R^9$ are as hereinbefore defined) by reaction with one or more reagents serving to replace the enolic hydroxy group by, or convert the enolic hydroxy group into, a leaving group e.g. a halogen atom or an acyloxy or ether group.

Compounds of formula (III) in which $R^6$ represents a halogen atom may, for example, be prepared by treating a compound of formula (IV) with dimethylformamide and phosphorus trichloride at a temperature of from -20° to +40°C.

Compounds of formula (III) in which $R^6$ is an acyloxy group may be prepared by reacting a compound of formula (IV) with an appropriate acylating agent, e.g. an acyl halide or anhydride. Such an acyl halide will desirably be a phosphinyl or sulphonyl halide, the phosphinyl and sulphonyl portions being as defined above. Thus, in this case, the compound of formula (IV) may be

reacted with an alkane sulphonyl halide or aryl sulphonyl halide, for example methanesulphonyl halide, p-toluenesulphonyl halide or a dialkoxyphosphinyl chloride e.g. dimethoxyphosphinyl chloride. Reaction is normally carried out in a solvent, e.g. an ether such as diethyl ether or tetrahydrofuran or a halogenated hydrocarbon such as methylene chloride. Reaction temperatures are generally from -30° to +30°C, preferably from -15° to +20°C.

The reaction will normally be effected in the presence of an acid binding agent, e.g. a tertiary amine such as triethylamine, pyridine or collidine or an oxirane such as propylene oxide.

Compounds of formula (III) in which $R^6$ is an ether group may, for example, be prepared from compounds of formula (IV) by reaction with a diazoalkane, e.g. diazomethane, optionally in the presence of a Lewis acid catalyst such as boron trifluoride etherate. The reaction may be effected in a solvent such as an ether e.g. diethyl ether, dioxan or tetrahydrofuran, a hydrocarbon e.g. light petroleum fraction, or a halogenated hydrocarbon e.g. dichloromethane or chloroform. The reaction temperature is preferably low, e.g. -15° to +15°C.

Alternatively a compound of formula (II) where $R^6$ is a leaving group, and $R^5$ represents a halogen atom may be prepared from a compound corresponding to formula (II) but where $R^6$ is hydroxy., by reaction with one or more reagents serving to replace the hydroxy group by or convert it into a leaving group, e.g. a halogen atom or an acyloxy or ether group. Such reagents may be of the type previously described in relation to compounds of formula (IV). For acylation an acid binding agent will

normally be present, e.g. an oxirane such as propylene oxide or a base; where a base is used, this is preferably a weak base such as pyridine.

The compounds corresponding to formula (II) in which $R^5$ is halogen but in which $R^6$ is hydroxy may be prepared from compounds of formula (IV) by reaction with an electrophilic halogenating agent e.g. of the type described above in relation to the compounds of formula (III).

The compounds of formula (IV) wherein $R^9$ represents a group of formula $-SR^{10}$ as defined above and $R^{1a}$ represents a grouping of formula $-CH_2-CH_2NHR^4$ or $CH_2CH_2R^c$ (where $R^4$ and $R^c$ are as hereinbefore defined), may be readily prepared from the compounds of formula (V)

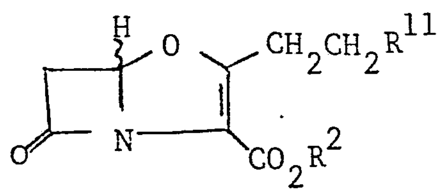

(V)

[wherein $R^2$ is as defined above and $R^{11}$ represents a grouping of formula $NHR^4$ or $R^c$ (where $R^4$ and $R^c$ are as defined above)] by reaction thereof with a thiol $R^{10}SH$, wherein $R^{10}$ is as defined above.

The thiol $R^{10}SH$ will preferably be present in excess as compared with the starting material of formula (V).

The reaction will desirably be carried out in an aprotic organic solvent, e.g. an ether solvent such as tetrahydrofuran although the thiol may itself serve as the medium for the reaction. The reaction will desirably be carried out at or above ambient temperature, e.g. at from +20° to +80°C.

The compounds of formula (V) may be prepared from compounds of formula (VI)

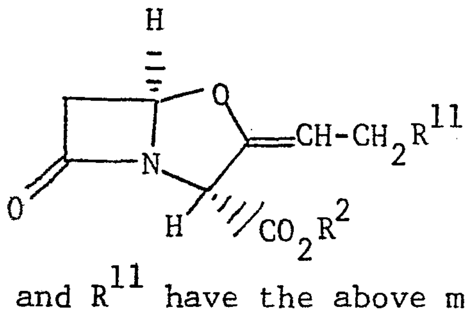

(VI)

(wherein $R^2$ and $R^{11}$ have the above meanings) analogously to the method described in Belgian Patent No. 858515 by treatment thereof at elevated temperature with a base. Preferred bases for use in this reaction are tertiary organic bases e.g. tertiary amines. Such amines will desirably have the formula $R^xR^yR^zN$, where $R^x$, $R^y$ and $R^z$ are as defined above.

Use of simpler trialkylamines e.g. having 1-6 carbon atoms in each alkyl group, particularly methyl, ethyl, propyl or butyl groups, is preferred and triethylamine is especially preferred.

Reaction will desirably be carried out in a suitable inert solvent. Such solvents will preferably have some degree of polarity and include esters e.g. ethyl acetate, ethers e.g. tetrahydrofuran, ketones e.g. acetone, amides e.g. dimethylformamide and halogenated hydrocarbons e.g. 1,2-dichloroethane or chloroform.

Reaction will desirably be effected under reflux, a temperature of from 50°C to 100°C being preferred.

Compounds of formula (II) wherein $R^5$ represents a group of formula $-\overset{\oplus}{N}R^xR^yR^z$ may be prepared by reaction of compounds of formula (VII)

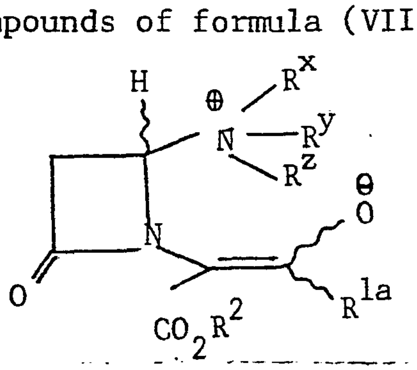

(VII)

BAD ORIGINAL

(where $R^{1a}$, $R^2$, $R^x$, $R^y$ and $R^z$ have the above meanings) by reaction with an appropriate reagent serving to introduce the group $R^6$. The introduction of the group $R^6$ may be effected by the procedures described above for the formation of compounds of formula (III). In particular, the compound of formula (VII) may be reacted with a sulphonylating agent e.g. a methanesulphonylating agent such as methane sulphonyl chloride to form a product of formula (II) in which $R^6$ is methanesulphonyloxy. In general, as mentioned above, an anion $A^{\ominus}$, derived from the reagent used for introduction of the group $R^6$, e.g. a halide ion such as a chloride ion will be associated with the compound of formula (II) thus formed. The reaction may be effected in the presence of a base for example a tertiary base such as pyridine. The latter may serve as solvent or a further solvent may be present, e.g. a halogenated hydrocarbon such as methylene chloride or 1,2-dichloroethane.

If desired such a compound of formula (VII) may be used in the presence of pyridine to prepare a compound of formula (II) in situ which compound may then be directly cyclised to a compound of formula (I) by addition of triethylamine and passing in hydrogen sulphide. Heating, e.g. to about 70°C completes the reaction. Where a compound of formula (IIA) is prepared from a compound of formula (VII) without isolation of the compound of formula (II), the compound of formula (IIA) may be partly in the form of an acid addition salt with an acid HA (where A is as defined above ) such as HCl.

Compounds of formula (VII) may, for example, be prepared by reaction of a compound corresponding to formula (II) (in which $R^5$ is halogen but in which $R^6$ is a hydroxy group) with an excess of a tertiary base $NR^xR^yR^z$ in the presence of a solvent such as ethyl acetate at temperatures of from 0 to 30°C, preferably at ambient temperature.

Compounds of formula (VII) in which $R^1$ represents a grouping of formula $-CH_2CH_2-R^{11}$ (where $R^{11}$ is as hereinbefore defined) may alternatively be prepared by a ring-opening reaction on a compound of formula (VI) employing a base $NR^xR^yR^z$. Where $NR^xR^yR^z$ is to be the residue of a weak base such as pyridine it may be possible to perform the ring-opening by reaction with the weak base in the presence of a catalytic quantity of a strong base. The ring-opening reaction is preferably effected in the presence of a solvent such as ethyl acetate at temperatures of from 0 to 30°C, most preferably at ambient temperature. Compounds of formula (VII) in which $R^1$ represents a grouping of formula $-CH_2CH_2R^{11}$ may also be prepared by treatment of a compound of formula (V) with a base $NR^xR^yR^z$.

Compounds of formula (VI) may be prepared as described in German OLS 27 47 599 or by methods analogous thereto.

It should be noted that in the above reaction sequences and those described hereinafter the groups $R^{1a}$ and $COOR^{2a}$ may be modified (using the above described techniques) between the various reaction stages set out, although remaining within the stated definition. Thus,

for example conversion of an azido group or protected amino group into a group of formula $-NHR^4$ as required in the final compound of formula (I), and/or deesterification of a group such as $R^{2a}$ followed by reesterification, may be carried out either on a compound of formula (VI), after the cyclisation of a compound of formula (II) or at some intermediate stage. Protecting groups on $R^{1a}$ may also be replaced by others which may be more appropriate at an intermediate stage.

The compounds of formula (IV) in which $R^{1a}$ represents a group $-R^3R^c$ in which $R^c$ is an azido group may be prepared as described in our European Patent Application No. 3415.

Certain of the compounds of formula (II), (IIa), (III) and (IV) and compounds corresponding to formula (II) in which $R^5$ is halogen but wherein $R^6$ is hydroxy are believed to be novel compounds and thus according to a further feature of the invention there are provided compounds of the general formula (VIII)

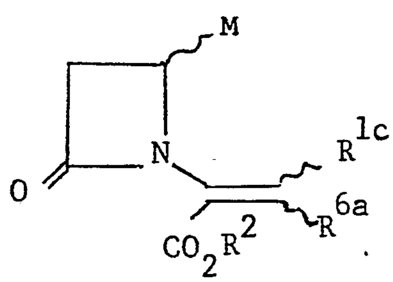

(VIII)

wherein $R^{1c}$ is as defined for $R^{1a}$ other than a group of formula $-R^3N_3$ (where $R^3$ is as defined above); $R^2$ represents a carboxyl esterifying group; and M either represents a halogen atom or a group $R^9$ ( in which $R^9$

represents a group $-SR^{10}$ or $-\overset{O}{S}-R^{10}$ where $R^{10}$ is an aliphatic, araliphatic, aromatic or heterocyclic group) and $R^{6a}$ represents a hydroxy group or a leaving group; or M represents a group of formula

$$\overset{\oplus}{N}R^xR^yR^zA^\ominus$$

(wherein $R^x$, $R^y$, $R^z$ and $A^\ominus$ are as hereinbefore defined) and $R^{6a}$ represents a leaving group; or M represents a group

$$\overset{\oplus}{N}R^xR^yR^z$$

(where $R^x$, $R^y$ and $R^z$ are as hereinbefore defined) and $R^{6a}$ represents $S^\ominus$ or $O^\ominus$. Compounds of general formula (V) (except where $R^{11}$ is a group $-R^3R^c$ where $R^c$ is $-N_3$) are also new and constitute a further feature of the invention.

Processes for the preparation of the compounds embraced by formula (VIII) (such processes being as set out hereinbefore) constitute yet further features of the present invention

The invention also includes non-toxic physiological precursors of the active compounds of the invention, that is substances which on administration are converted in vivo into the antibiotic compounds. In the case of the acids of formula (I) ($R^{2a}$=H), their metabolically labile esters (which already fall within the definition of the compounds of the invention) are examples of such physiological precursors.

The foregoing reactions are illustrated in the following diagram:

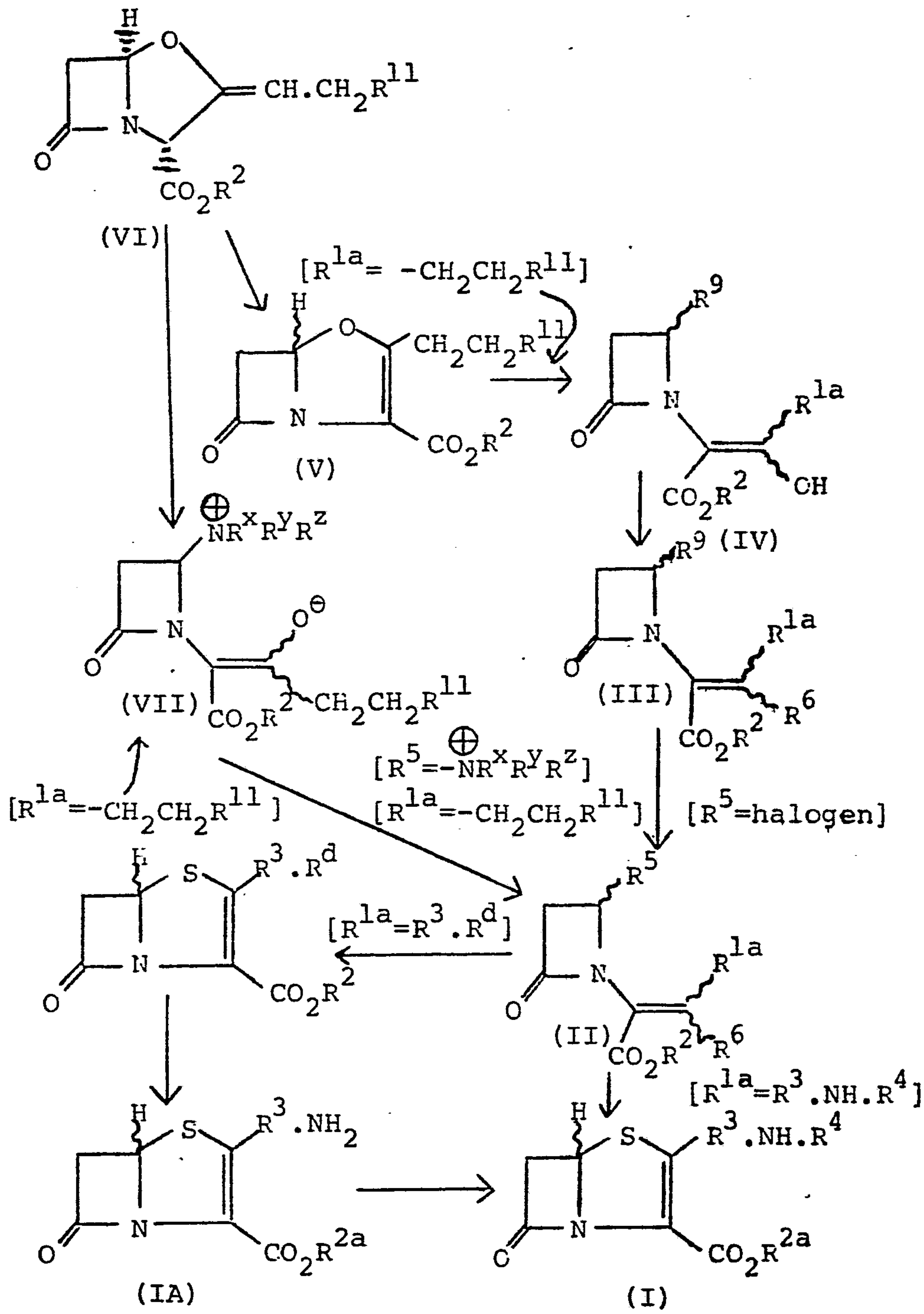
(VI)
(V)
(IV)
(VII)
(III)
(II)
[$R^{1a}$ = $-CH_2CH_2R^{11}$]
[$R^5$=$-\overset{\oplus}{N}R^xR^yR^z$]
[$R^{1a}$=$-CH_2CH_2R^{11}$]
[$R^5$=halogen]
[$R^{1a}$=$-CH_2CH_2R^{11}$]
[$R^{1a}$=$R^3.R^d$]
[$R^{1a}$=$R^3.NH.R^4$]

(IA) [B = 〉S]

(I) [B = 〉S]

The following non-limiting Examples serve to illustrate compounds of the present invention and processes for their preparation.

In the examples, certain bicyclic compound are named with reference to "penam", the conventional name given to the heterocycle of formula (XIV),

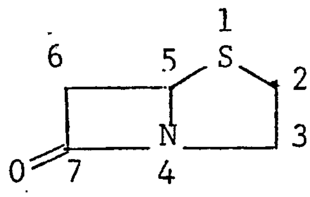

(XIV)

or "clavam", the name given to the heterocycle of formula (XV),

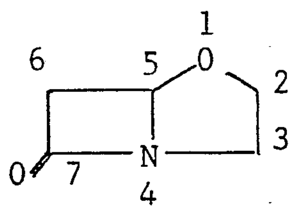

(XV)

Example 1

4-Azido-1-(4-nitrobenzyloxycarbonyl)-1-(2-oxo-4-triethylammonioazetidin-1-yl)but-1-en-1-olate

A solution of 4-nitrobenzyl (3R,5R,Z)-2-(2-azidoethylidene)-clavam-3-carboxylate (0.377 g) and triethylamine (0.30 ml) in ethyl acetate (3.5 ml) was stood at 28° for 40 minutes. The resulting solid was collected and dried *in vacuo* to give the title salt (0.092 g), $\nu_{max}$ (Nujol) 2100 ($N_3$), 1765 and 1750 (β-lactam), 1652 ($CO_2R$), 1520 and 1348 $cm^{-1}$ ($NO_2$), τ (DMSO-$d_6$) values for *ca*. 1:1 mixture of isomers include 1.77 (t, J 8Hz, aromatic protons), 2.38 + 2.40 (d, J 8Hz, aromatic protons), 4.46 + 4.63 (m, azetidinyl C-4 H), 4.84 + 4.73 and 4.96 (s + ABq, J 14Hz, benzylic protons), 8.80 + 8.88 (t, J 7Hz, $N(CH_2CH_3)_3$).

Example 2

4-Nitrobenzyl(5RS)-2-(2-azidoethyl)pen-2-3m-3-carboxylate

Methanesulphonyl chloride (2.12 g) was added to a stirred suspension of 4-azido-1-(4-nitrobenzyloxycarbonyl)-1-(2-oxo-4-triethylammonioazetidin-1-yl)but-1-en-2-olate (2.846 g) in dry 1,2-dichloroethane (90 ml) containing pyridine at 5°. The reaction mixture was allowed to warm to room temperature and after 35 minutes was cooled to 5°. Hydrogen sulphide was passed through the stirred solution under nitrogen, and an excess of triethylamine was added in portions over 10 minutes. The resulting mixture was diluted with 1,2-dichloroethane (100 ml) and heated to boiling for 5 minutes. The solution was cooled to ambient temperature and washed with saturated brine containing 0.5 N aqueous hydrochloric acid (twice),

followed by saturated brine, and then dried over magnesium sulphate. The filtered solution was evaporated to low volume, silica gel was added, and the resulting slurry was evaporated to dryness. The impregnated silica gel was placed on top of a dry silica gel comumn and eluted with ether-petroleum ether (40-60°) mixture. Appropriate fractions were combined and evaporated to afford the title ester (1.36 g) $\lambda_{max}$ ($CHCl_3$) 318nm (ε 6500), $\nu_{max}$ ($CHBr_3$) 2100 ($N_3$), 1786 (β-lactam), 1705 (ester), 1518 and 1344 $cm^{-1}$ ($NO_2$), τ ($CDCl_3$) 1.78 and 2.38 (doublets, J 9 Hz, aromatic protons), 4.31 (dd, J 4 and 2Hz, C-5 H), 4.54 and 4.78 (ABq, J 14 Hz, benzylic protons), 6.15 and 6.49 (dd, J 16 and 4 Hz, and dd, J 16 and 2 Hz, C-6 protons), 6.50 (m, $-C\underline{H}_2N_3$), 6.6 to 7.2 (m, $-C\underline{H}_2CH_2$).

Example 3

(5RS)-2-(2-Aminoethyl)pen-2-em-3-carboxylic acid

A solution of 4-nitrobenzyl (5RS)-2-(2-azidoethyl)-pen-2-em-3-carboxylate (1.15 g) in ethyl acetate: ethanol (1:1; 50 ml) containing 10% palladium on charcoal (6.9 g) was shaken and hydrogenated at room temperature and at 1 atmosphere pressure. After 40 minutes the mixture was diluted with water (40 ml) and shaken for 5 minutes. The mixture was filtered through kieselguhr and the pad was washed with a little water followed by ethyl acetate. The aqueous phase was rewashed with ethyl acetate and then freeze-dried. The resulting foam was dissolved in water (2 ml) and, with stirring, was diluted with acetone (50 ml).

The resulting solid was washed with acetone and then collected and dried *in vacuo* to afford the *title acid* (0.103 g), $\lambda_{max}$ (pH 6 buffer) 303 nm ($\varepsilon$ 4000), $\nu_{max}$ (Nujol) 1753 ($\beta$-lactam), 1580 $cm^{-1}$ ($CO_2^-$), $\tau$ ($D_2O$ + DMSO-$d_6$) 4.30 (dd, J 3 and 1 Hz, C-5 H), 6.20 and 6.53 (dd, J 16 and 3 Hz, and dd, J 16 and 1 Hz, C-6 protons), 6.82 (m, $-C\underline{H}_2NH_2$), 6.8 to 7.2 (m, $-C\underline{H}_2CH_2$).

Example 4

(5RS)-2-(2-Formamidoethyl)pen-2-em-3-carboxylic acid

Bis-(tri-n-butyltin) oxide (0.55 ml) was added to a stirred suspension of (5RS)-2-(2-aminoethyl)pen-2-em-3-carboxylic acid (0.445 g) and 4-nitrophenylformate (0.347 g) in dry N,N-dimethylformamide (4 ml) at ambient temperature. After 60 minutes the solution was diluted with ethyl acetate (200 ml) and extracted (twice) with saturated brine (30 ml followed by 10 ml). The aqueous phase was extracted with ethyl acetate (2 x 200 ml), then adjusted to pH 5.0, and re-extracted with ethyl acetate (2 x 200 ml). The aqueous phase was saturated with ammonium sulphate, then covered with ethyl acetate (200 ml) and, with stirring, was acidified to pH 1.5. The aqueous phase was separated and re-extracted with ethyl acetate (3 x 200 ml). The combined organic extracts were dried over magnesium sulphate and then evaporated to low volume to give a slurry of crystals which was collected and dried *in vacuo* to afford the *title acid* (0.176 g), $\lambda_{max}$ (pH 6 buffer) 303 nm ($\varepsilon$ 6,060), $\nu_{max}$ (Nujol) 3350 (NH), 2500 and 1640 ($CO_2H$), 1776 ($\beta$-lactam), 1680 and 1532 $cm^{-1}$ (CONH, $\tau$ ($d_6$-DMSO)-2.6 br

(s, $CO_2H$), 2.08 br (s, NH), 2.18 (s, CHO), 4.46 (dd, J 4 and 2 Hz, C-5 H), 6.25 and 6.64 (dd, J 15 and 4 Hz; and dd, J 15 and 2 Hz, C-6 protons), 6.7 to 6.9 (m, $CH_2C\underline{H}_2NH$), 6.9 to 7.4 (m, $C\underline{H}_2CH_2NH$).

Example 5

1-Benzyloxycarbonyl-4-formamido-1-(2-oxo-4-triethylammonioazetidin-1-yl)but-1-en-2-olate

Triethylamine (2.59 ml) was added to a stirred solution of benzyl (3R,5R,Z)-2-(2-formamidoethylidene)-clavam-3-carboxylate (2.934 g) in ethyl acetate (25 ml) at 5°. The solution was allowed to warm to ambient temperature and was stirred for 2.5 hours after which the supernatant was decanted from the deposited oil. Trituration of the oil with ether gave a solid which was collected, washed with ether, and dried *in vacuo* to afford the *title betaine* (2.373 g), $\lambda_{max}$ (pH 6 buffer) 272.5 nm ($\varepsilon$ 15,100), $\nu_{max}$ (Nujol) 1764 ($\beta$-lactam), 1660 ($CO_2R$), 1660 and 1510 $cm^{-1}$ (COHN), $\tau$ ($D_6$-DMSO) values include for mixture of isomers 2.06 (s, CHO), 2.26 br (s, NH), 2.68 (s, aromatic protons), 4.57 + 4.9 (multiplets, azetidinyl C-4 proton), 4.8 to 5.3 (m, benzylic protons), 8.92 (m, $N(CH_2C\underline{H}_3)_3$).

Example 6

Benzyl (5RS)-2-(2-formamidoethyl)pen-2-em-3-carboxylate

Methanesulphonyl chloride (1.41 ml) was added to a stirred solution of 1-benzyloxycarbonyl-4-formamido-1-(2-oxo-4-triethylammonioazetidin-1-yl)but-1-en-2-olate (2.536 g) in dry 1,2-dichloroethane (40 ml) containing dry pyridine (2.46 ml) and maintained at 5°.

After 5 minutes the solution was allowed to warm to room temperature and stirred for 25 minutes. Hydrogen sulphide was passed through the stirred solution under nitrogen at 0°, and an excess of triethylamine was added over 5 minutes. The resulting mixture was subjected to a fast stream of nitrogen, and, after diluting with 1,2-dichloroethane, was heated to boiling for 3 minutes. The mixture was cooled to ca. 10°, then washed successively with 0.5 N aqueous hydrochloric acid (twice), 0.5 N aqueous sodium hydrogen carbonate (twice), water and saturated brine, and dried over magnesium sulphate. The solution was evaporated to low volume, silica gel was added, and the mixture was evaporated to dryness. The impregnated silica gel was transferred to the top of a dry silica gel column and fractionated eluting with ethyl acetate-petroleum ether (40-60°) mixtures. Appropriate fractions were combined and evaporated to afford the title ester (0.775 g) as an oil, $\lambda_{max}$ ($CHCl_3$) 317.5 nm (ε 5,850), $\nu_{max}$ ($CHBr_3$)3420 (NH), 1790 (β-lactam), 1700 ($CO_2R$), 1684 and 1506 (CONH), 1580 $cm^{-1}$(C=C), τ ($CDCl_3$) 1.76 (s, CHO), 2.64 (s, aromatic protons), 4.02 br (s, NH), 4.40 (dd, J 4 and 2 Hz, C-5 H), 4.78 (s, benzylic protons), 6.20 and 6.54 (dd, J 16 and 4 Hz; and dd, J 16 and 2 Hz, C-6 protons), 6.4 to 6.7 (m, $CH_2C\underline{H}_2NH$), 6.8 to 7.3 (m, $CH_2CH_2NH$).

Example 7

Potassium (5RS)-2-(2-acetamidoethyl)pen-2-em-3-carboxylate

A stirred suspension of (5RS)-2-(2-aminoethyl)-pen-2-em-3-carboxylic acid (0.059 g)in dry N,N-dimethylformamide (2 ml) was treated with bis-(tri-n-butyltin)-oxide (0.08 ml) followed by acetic anhydride (0.03 ml) at room temperature. After 25 minutes the solution was partitioned between ethyl acetate (100 ml) and saturated aqueous ammonium sulphate solution (20 ml) containing sodium hydrogen carbonate (0.36 g). The aqueous phase was washed with ethyl acetate (2 x 100 ml), then acidified with 2N aqueous hydrochloric acid to pH 1.0, resaturated with ammonium sulphate, and extracted with ethyl acetate (3 x 100 ml). The combined organic extracts were dried over magnesium sulphate, then evaporated to low volume (ca. 5 ml) and treated with an ethyl acetate solution of potassium 2-ethylhexanoate (0.674 M, 0.25 ml). Dilution with ether gave a deliquescent solid which was collected to afford the title salt (0.005 g) and then redissolved in deuterium oxide (0.45 ml), τ ($D_2O$) values include 4.38 (dd, J 4 and 2 Hz, C-5 H), 6.21 (dd, J 17 and 4 Hz, C-6 proton), 6.4 to 6.8 (m, C-6 proton and $-C\underline{H}_2CH_2NH-$) 6.8 to 7.3 (m, $-CH_2C\underline{H}_2NH-$), 8.05 (s, $NHCOC\underline{H}_3$).

Example 8

Benzyl (5RS)-2-(2-formamidoethyl)pen-2-em-3-carboxylate

A solution of benzyl (5RS)-2-(2-azidoethyl)-pen-2-em-3-carboxylate (0.268 g) in ethyl acetate-ethanol (1:1, 40 ml) containing 10% palladium on characoal (0.805 g) was shaken and hydrogenated at

room temperature and 1 atmosphere pressure. After 35 minutes the mixture was filtered through kieselguhr, and the pad was washed with a little ethyl acetate-ethanol (1:1). The combined filtrates were evaporated to low volume and, with stirring, were treated with bis-(tri-n-butyltin)oxide (0.22 ml) followed by a solution of 4-nitrophenylformate (0.136 g) in ethyl acetate (2 ml). After 5 minutes the soltuion was diluted with ethyl acetate and washed with water. The aqueous phase was extracted with ethyl acetate and the combined organic solutions were dried over magnesium sulphate, then evaporated to dryness onto silica gel. The impregnated silica gel was transferred to the top of a silica gel column and eluted with ether, ethyl acetate, and acetone:ethyl acetate (1:1). The latter fractions were combined and evaporated to leave the title ester (0.061 g). Spectroscopic data resembled those as described in Example 6.

Example 9

Potassium (5RS)-2-(2-acetamidoethyl)pen-2-em-3-carboxylate

A stirred suspension of (5RS)-2-(2-aminoethyl)-pen-2-em-3-carboxylic acid (0.250 g) in dry N,N-dimethylformamide (8.5 ml) was treated with bis-(tri-n-butyltin)oxide (1.0 ml) followed by acetic anhydride (0.13 ml) at room temperature. After 60 minutes the solution was partitioned between ethyl acetate (200 ml) and 0.05 M aqueous sodium hydrogen carbonate (2 x 25 ml). The aqueous phase was washed with ethyl acetate (2 x 200 ml) then acidified with 2 N aqueous hydrochloric acid to

pH 1.0, saturated with ammonium sulphate, and extracted with ethyl acetate (3 x 200 ml). The combined organic extracts were dried over magnesium sulphate, then evaporated to low volume (ca. 5 ml) and treated with an ethyl acetate solution of potassium 2-ethylhexanoate (0.674 M, 0.87 ml). Dilution with ether gave a deliquescent solid which was collected to afford the title salt (0.036 g), $\lambda_{max}$ (pH 6 buffer) 257 ($\varepsilon$ 3,530), and 301 mn ($\varepsilon$ 3,890), $\nu_{max}$ (Nujol), 1770 ($\beta$-lactam), 1666 (CONH), 1590 $cm^{-1}$ ($CO_2^-$).

Example 10

Potassium (5RS)-2-(2-N-methylureidoethyl)pen-2-em-3-carboxylate

A suspension of 2-(2-aminoethyl)pen-2-em-3-carboxylic acid (0.150 g) in dry N,N-dimethylformamide (3 ml) was treated with bis (tri-n-butyltin)oxide (0.35 ml) followed by methyl isocyanate (0.08 g). After 30 minutes the solution was diluted with ethy; acetate and extracted (twice) with 0.024 M aqueous potassium bicarbonate (2 x 15 ml). The aqeuous phase was treated with charcoal, filtered and freeze-dried. The resulting foam was titturated with ether and acetone to afford a solid which was collected and dried in vacuo to give the title salt (0.150 g), $\lambda_{max}$ (pH 6 buffer) 257 ($\varepsilon$ 2,660), and 302 nm ($\varepsilon$ 3,400), $\nu_{max}$ (Nujol) 3320 (NH) ($\beta$-lactam), 1650 (CONH), and 1588 $cm^{-1}$ ($CO_2^-$).

Example 11

Potassium (5RS)-2-(2-N-phenylureidoethyl)pen-2-em-3-carboxylate

A suspension of (5RS)-2-(2-aminoethyl)pen-2-em-3-carboxylic acid (0.150 g) in dry N,N-dimethylformamide (3 ml) was treated with bis-(tri-*n*-butyltin)-oxide (0.35 ml), then cooled to 0°, and treated with phenyl isocyanate (0.167 g). The xixture was allowed to warm to ambient temperature and after 60 minutes was diluted with ethyl acetate (300 ml) and extracted with 1.0 N aqueous sodium hydrogen carbonate (2 x 25 ml). The aqueous extracts were acidified to pH 1.0 under ethyl acetate (200 ml). The aqueous phase was further extracted with ethyl acetate (2 x 250 ml) and the combined organic extracts were dried over magnesium sulphate. The filtered organic solution was concentrated to *ca*. 50 ml, diluted with ethanol (50 ml), and treated with a solution of potassium 2-ethylhexanoate in ethyl acetate (0.674 M, 0.62 ml). The resulting cloudy solution was concentrated to *ca*. 20 ml, diluted with ether, and the deposited solid was collected and dried *in vacuo* to afford the *title salt* (0.125 g), $\lambda_{max}$ (pH 6 buffer) 240 (ε 16,500 and 302 nm (ε4,680), $\nu_{max}$ (Nujol) 3310 (NH), 1776 (β-lactam), 1628 and 1460 (CONH), 1590 ($CO_2^-$), 1560 $cm^{-1}$ (C=C).

Example 12

p-Nitrobenzyl (4'RS)-4-azido-3-hydroxy-2-(2'-oxo-4'-p-tolylthioazetidin-1'-yl)but-2-enoate

A stirred and cooled (0°) suspension of potassium azidoacetate (0.164 g) in dry ether (3 ml) was treated successively with oxalyl chloride (0.1 ml) and a drop of dry N,N-dimethylformamide. The suspension was stirred at 0° for 40 minutes.

n-Butyl lithium in hexane (1.52 molar solution, 1.15 ml) was added dropwise under nitrogen to a cooled (-78°) solution of hexamethyldisilazane (0.37 ml) in dry tetrahydrofuran (10 ml). A solution of p-nitrobenzyl (4'RS)-2-(2'-oxo-4'-p-tolylthioazetidin-1'-yl) acetate (0.386 g) in dry tetrahydrofuran (10 ml) was then added followed, after 2 minutes, by the solution of azidoacetyl chloride described above. After stirring for 20 minutes at -78° the mixture was poured into a mixture of ethyl acetate and 0.5 N hydrochloric acid (100 ml of each).

The organic layer was washed with water, and brine (40 ml of each) and dried and the solvent was removed. The residue was purified by preparative layer chromatography using dichloromethane:acetone (20:1) for development.

The appropriate band was removed and extracted into acetone (100 ml) and the extract was evaporated to dryness. The residue was suspended in ether (20 ml), the suspension was stirred for 5 minutes and the insoluble material was filtered off, and washed with ether (2 x 5 ml), and dried to give the title ester (0.107 g) as a solid m.p 236° (decomp), $\lambda_{max}$ (EtOH) 277 nm ($E^{1\%}_{1cm}$ 470) with an inflection at 262 nm ($E^{1\%}_{1cm}$ 417) and $\gamma_{max}$ (Nujol) 2135 and 2105 (azide), 1748 $cm^{-1}$ (β-lactam).

Example 13

p-Nitrobenzyl (2'RS)-4-azido-3-methanesulphonyloxy-2-(2'-chloro-4'-oxoazetidin-1'-yl)but-2-enoate

A stirred and cooled (0°) solution of the product of Example 11 (1.36 g ) in dry dichloromethane was treated successively with methanesulphonyl chloride (0.34 ml) and triethylamine (0.60 ml).

After 20 minutes 0.5N hydrochloric acid (30ml) was added and the mixture was stirred for a further 5 minutes. The organic phase was separated, washed with water, and brine (20 ml of each) and dried.

A solution of chlorine in dichloromethane (8.2 ml of a 5% solution, equivalent to 5.8 mmole of chlorine) was added to the above cooled (0°) solution.

The mixture was stirred at 0° for 20 minutes, the solvent was evaporated off and the residue was purified by column chromatography on kieselgel (30g). The column was eluted with toluene; ethyl acetate (20:1) and the appropriate fractions were combined and the solvent removed to give the title compound (0.548 g) as a gum, $\lambda_{max}$ ($CHCl_3$) 262.5nm ($E^{1\%}_{1cm}$ 342) and $\nu_{max}$ ($CHBr_3$) 2100 (azide), 1790 $cm^{-1}$ (β-lactam).

Example 14

p-Nitrobenzyl (5RS)-2-azidomethyl-pen-2-em-3-carboxylate

Hydrogen sulphide (1 ml of a 1.4 molar solution in N,N-dimethylformamide) was added to a stirred and cooled (-20°) solution of triethylamine (0.26 ml) in dry N,N-dimethylformamide (20 ml), under nitrogen.

A few minutes later a cooled (-20°) solution of the product of Example12 (0.43 g) in dry N,N-dimethylformamide (20 ml) was added and the mixture was stirred for a further 20 minutes at -20°. The reaction mixture was poured into a stirred mixture of ethyl acetate and N hydrochloric acid (40 ml of each), and the organic layer was separated and washed with water, and brine (20 ml of each). The organic solution was dried and the solvent removed to give an orange oil which was triturated with ethyl acetate: ether (1:5) (10 ml). The precipitate was filtered off and discarded. The filtrate was evaporated to an orange oil which was partitioned between ethyl acetate and N hydrochloric acid (100 ml of each). The organic phase was separated, and washed with water, and brine (30 ml of each) and dried and the solvent removed to give an orange foam (0.2 g).

This foam was subjected to preparative layer chromatography using toluene:ethyl acetate (4:1), for development. The appropriate band was extracted into ethyl acetate (50 ml) and the extract was evaporated to dryness to give the title compound (0.03 g) as a gum; $\lambda_{max}$ ($CHCl_3$) 269.5 nm ($E^{1\%}_{1cm}$ 417) with an inflection at 320 nm ($E^{1\%}_{1cm}$ 128) and $\nu_{max}$ ($CHBr_3$) 2110 (azide), 1788 $cm^{-1}$ (β-lactam).

The p-nitrobenzyl (5RS)-2-azidomethylpen-2-em-3-carboxylate may then be converted by the foregoing methods into corresponding 2-acylaminomethyl compounds.

The following Examples illustrate how compounds according to the invention maybe formulated into pharmaceutical compositions

Example A

Formula per tablet

| | |
|---|---|
| Densified potassium (5RS)-2-(2-formamidoethyl-pen-2-em-3-carboxylate containing 1% magnesium stearate | 302.0 mg |
| Empicol LZ | 4.5 mg |
| Explotab | 9.0 mg |
| Avicel PH 101 to tablet core weight of | 450.0 mg |

Method of Preparation

Blend the antibiotic with magnesium stearate and compress into slugs on a heavy duty tableting machine. Break down the slugs through a series of screens (10, 12, 16 and 20 mesh) on a rotary granulator to produce free-flowing granules. Blend the granules with the rest of the excipients and compress the blend on a tablet machine using normal or deep concave punches (9 - 12 mm diameter).

Film coat the tablet cores using hydroxypropylmethyl cellulose or similar film forming material using either an aqueous or non-aqueous solvent system. A plasticizer and suitable colour may be included in the film coating solution.

Example B

Formula per tablet

| | |
|---|---|
| Potassium (5RS)-2-(2-formamidoethyl)-pen-2-em-3-carboxylate | 299.0 mg |

| | |
|---|---|
| Maize starch | 22.5 mg |
| Explotab | 9.0 mg |
| Empicol LZ | 4.5 mg |
| Magnesium stearate | 4.5 mg |
| Avicel PH 101 to tablet core weight of | 450.0 mg |

Method of Preparation

Add sufficient quantity of a 10% starch paste to the antibiotic to produce a suitable wet mass for granulation. Prepare the granules and dry using a tray or fluid bed dryer. Sift through a sieve (10 or 12 mesh), add the remaining ingredients and compress into tablets as described in Example A.

The tablet cores may be film coated as described in Example A.

Example C

Formula per capsule

| | |
|---|---|
| Densified potassium (5RS)-2-(2-formamidoethyl)pen-2-em-3-carboxylate containing 1% magnesium stearate | 302.0 mg |
| Explotab | 6.0 mg |
| Aerosil 200 | 2.0 mg |

Method of Preparation

Densify and prepare granules of the antibiotic as described in Example A, blend the granules and excipients and fill the blend into appropriate size hard gelatin capsules (lock fitting type) on an automatic capsule filling machine.

Example D

Dry Powder for Injection

Fill sterile potassium (5RS)-2-(2-formamidoethyl)-pen-2-em-3-carboxylate aseptically into glass vials under a blanket of sterile nitrogen such that each vial contains an amount equivalent to 500 mg of the antibiotic free acid. Close the vials using rubber discs or plugs held in position by aluminium sealing rings, thereby preventing gaseous exchange or ingress of microorganisms. Constitute the product by dissolving in Water for Injections shortly before administration.

Empicol LZ is sodium lauryl sulphate available from Marchon Ltd.; Explotab is sodium starch glycolate available from Greeff Fine Chemicals Ltd., Croydon, Surrey, England; Avicel PH 101 is microcrystalline cellulose available from FMC Corporation, U.S.A.; Emcompress is dicalcium phosphate dihydrate NF; and Aerosil 200 is finely divided silicon dioxide.

Results of Biological Tests

Biological testing of a number of compounds of the invention has provided the results shown below; the test compounds are identified by their Example Numbers:

Determination of Minimum Inhibitory Concentrations (MIC)

Serial two-fold dilutions of freshly prepared test solutions were made into Oxoid No. 1 Nutrient agar with or without added enrichment and poured into petri dishes. Plates were inoculated with a multipoint inoculator with inoculum containing approximately

$10^5$ colony forming units of the organisms shown in the following Table, all of which organisms are clinical isolates. The MIC, quoted in the Table as µg/ml, was read after 18 hours incubation at 37°C as the lowest concentration which inhibited growth.

| Organism / Example No: | | 4 | 11 | 10 | 7 |
|---|---|---|---|---|---|
| Staph. aureus | 853E | 2 | 0.2 | 62 | 2 |
| Micrococcus Sp | 1810E | 0.1 | 0.2 | 1 | 2 |
| E. coli | 851E | 1 | 2 | 8 | 8 |
| E. cloacae | 1051E | 1 | 16 | 16 | 16 |
| E. cloacae | 1321E | 1 | 8 | 8 | 4 |
| K. aerogenes | 1522E | 2 | 2 | 8 | 8 |
| S. marcescens | 1324E | 2 | 8 | 8 | 31 |
| H. influenzae | 1184E | 2 | 16 | 8 | 16 |

## Claims

1. Compounds of general formula

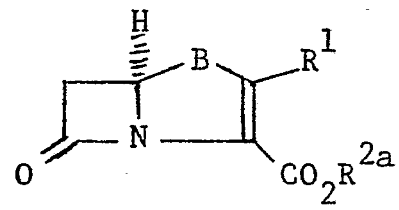

(I)

wherein $R^1$ represents a grouping of formula $-R^3.NH.R^4$ [where $R^3$ represents a group $-C_n H_{2n}-$ where n is an integer from 1 to 6 and $R^4$ represents an acyl group of formula $-CYR^a$, $-CY.ZR^b$ or $-CY.NHR^a$ (in which Y and Z, which may be the same or different, each represents oxygen or sulphur); $R^a$ represents a hydrogen atom; or an alkyl group which contains from 1 to 4 carbon atoms, optional substituents being selected from one or more of halogen, cyano, azido, amino, $C_{1-4}$ alkylthio, hydroxy, $C_{1-4}$ alkoxy and thiol; or an aralkyl group having up to 10 carbon atoms, optional substituents on the phenyl group when the aralkyl group is a benzyl group being selected from hydroxy or sulphonamido, optional substituents on the alkyl portion being selected from azido, amino, carboxyl or hydroxy;

or an aryl group having up to 10 carbon atoms, optional substituents when the aryl group is a phenyl group being selected from halogen, cyano, $C_{1-4}$ alkoxy or $C_{1-4}$ alkyl;

and $R^b$ represents a methyl or ethyl group)];

$R^{2a}$ represents a carboxyl esterifying group or a

hydrogen atom;

and B represents a group $\rangle$S or $\rangle$S→O ; and salts thereof.

2. Compounds as claimed in claim 1 wherein n is from 1 to 4, and B is a group $\rangle$S.

3. Compounds as claimed in claim 1 or claim 2 wherein n is 2 .

4. Compounds as claimed in any of claims 1 to 3 wherein $R^1$ represents a formamido, acetamido, ureido, methylureido or phenylureido group.

5. (5RS)-2-(2-Formamidoethyl)pen-2-em-3-carboxylic acid and salts thereof.

6. A process for the preparation of compounds of formula (I) which comprises reacting a compound of the formula (II)

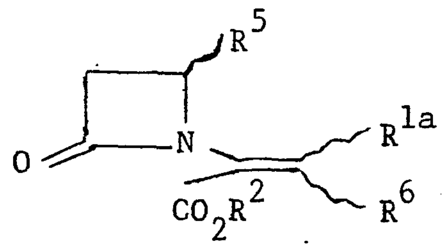

(II)

[wherein

$R^{1a}$ is as defined above for $R^1$ (except that any amino, carboxyl, hydroxy or thiol groups should be in protected from) or represents a group of formula $-R^3.R^c$ (where $R^3$ is as defined above and $R^c$ represents an azido or protected amino group);

$R^2$ represents a carboxyl esterifying group as defined above for $R^{2a}$;

$R^5$ represents a halogen atom or a group of formula $-NR^xR^yR^z$ (in which $R^x$, $R^y$ and $R^z$, which may be the same or different, each represents an aliphatic, araliphatic or

aromatic group or two of $R^x$, $R^y$ and $R^z$ together with the nitrogen atom to which they are attached represent a five-, six- or seven-membered heterocyclic ring optionally containing a further heteroatom and the third of $R^x$, $R^y$ and $R^z$ is as defined above or $R^x$, $R^y$ and $R^z$ together with the nitrogen atom to which they are attached form an aromatic heterocyclic ring or an aromatic or nonaromatic polycyclic heterocyclic system);

and $R^6$ represents a leaving group, for example a halogen atom or an acylated or etherified hydroxy group group] with hydrogen sulphide (where necessary in the presence of a base) or with a hydrosulphide or sulphide salt, followed where $R^{1a}$ is different from $R^1$ in the desired compound of formula (I) by conversion to a group of formula $-R^3NH_2$ (where $R^3$ is as hereinbefore defined) and subsequent acylation to give the desired compound of formula (I) deesterification being optionally effected before, during or after said conversion of a group $R^{1a}$ into a group $-R^3NH_2$; if desired, subsequently converting a compound of formula (I) in which B is $>S$ into a corresponding compound wherein B is $S\rightarrow O$; and/or if desired, converting the compound of formula (I) into a salt thereof; and/or, if desired, converting a compound of formula (I) in which $R^{2a}$ represents a hydrogen atom, or a salt thereof, into a corresponding ester, and/or, if desired, separating a mixture of 5R-and 5S-isomers to produce an individual 5R-isomer.

7. A process for the preparation of compounds of formula (I) which comprises reacting a compound of formula (IA)

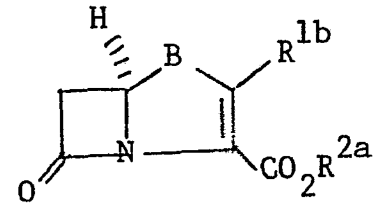

(IA)

(wherein $R^{1b}$ is a grouping of formula $-R^3NH_2$ where $R^3$ is as defined in claim 1 and $R^{2a}$ and B are as defined in claim 1) with an acylating agent, and/or if desired, converting the compound of formula (I) into a salt thereof; and/or, if desired, converting a compound of formula (I) in which $R^{2a}$ represents a hydrogen atom, or a salt thereof, into a corresponding ester and/or, if desired where the 5R-isomer of formula (IA) is admixed with corresponding 5S-isomers, separating the mixture of isomers so produced to yield an individual 5R-isomer.

8. A process as claimed in claim 7 wherein acylation is carried out in the presence of a bis-trialkyl tin oxide.

9. Pharmaceutical compositions comprising a compound of formula (I) as defined in claim 2 in which $R^{2a}$ represents a hydrogen atom or a metabolically labile carboxyl esterifying group or a salt of such a compound of formula (I) in association with a pharmaceutical carrier or excipient.

10. Compounds of the general formula (VIII),

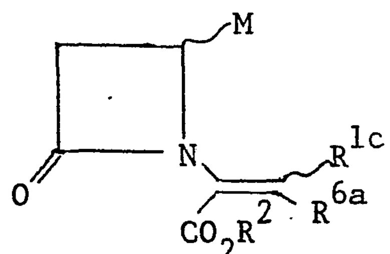

(VIII)

wherein $R^{1c}$ is as defined for $R^{1a}$ in claim 5 other than a group of formula $-R^3N_3$ where $R^3$ is as defined

in claim 1; $R^2$ represents a carboxyl esterifying group; and either M represents a halogen atom or a group $R^9$ (in which $R^9$ represents a group $-SR^{10}$ or a group

$$-\overset{\overset{O}{\uparrow}}{S}-R^{10}$$

where $R^{10}$ is an aliphatic, araliphatic, aromatic or heterocyclic group; and $R^{6a}$ represents a hydroxyl group or a leaving group; or M represents a group of formula

$$-\overset{\oplus}{N}R^xR^yR^z$$

(where $R^x$, $R^y$ and $R^z$ are as defined in claim 6) and $R^{6a}$ represents a leaving group; or Z represents a group

$$-\overset{\oplus}{N}R^xR^yR^z$$

(where $R^x$, $R^y$ and $R^z$ are as defined in claim 6) and $R^{6a}$ represents $S^{\ominus}$ or $O^{\ominus}$.

11. A process for the preparation of compounds of formula

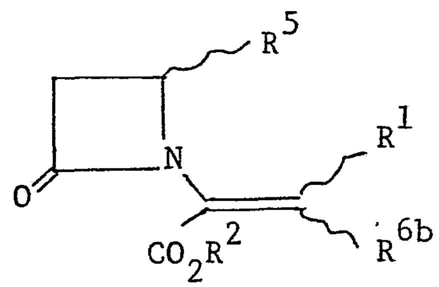

wherein $R^1$ is as defined in claim 1, $R^2$ represents a carboxyl esterifying group, $R^5$ represents a halogen atom and $R^{6b}$ represents a leaving group or a hydroxyl

group which comprises reacting a compound of formula

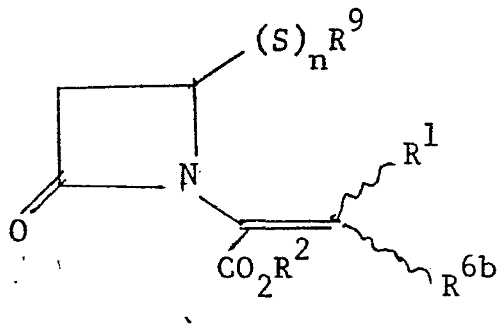

(wherein $R^1$ is as defined in claim 1, $R^2$ represents a carboxyl esterifying group, $R^{6b}$ is as defined above and n and $R^9$ are as defined in claim 9, with the proviso that when n is 1, $R^1$ represents a methyl group) with an electrophilic halogenating agent.

12. Compounds of the formula (V),

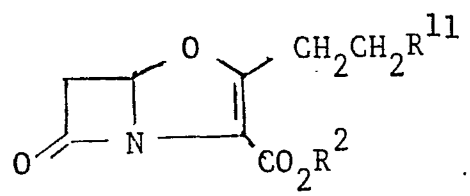

(V)

wherein $R^2$ represents a carboxyl esterifying group and $R^{11}$ represents a grouping of formula $-NHR^4$ or $R^c$ wherein $R^4$ is as defined in claim 1 and $R^c$ is a protected amino group.

European Patent Office

# EUROPEAN SEARCH REPORT

0010358

Application number

EP 79301944.9

| Category | Citation of document with indication, where appropriate, of relevant passages | Relevant to claim |
|---|---|---|
| P | DE - A1 - 2 819 655 (CIBA-GEIGY)<br>+ Claims 2 - 25, especially 16, 17 +<br>-- | 1-5,9 |
| P | EP - A1 - 0 000 636 (GLAXO)<br>+ Claims 1 - 8 +<br>-- | 1-10 |
| | DE - A1 - 2 740 527 (GLAXO)<br>+ Claims 1, 15 - 20 +<br>---- | 10, 12 |

CLASSIFICATION OF THE APPLICATION (Int. Cl. 3)

C 07 D 499/00
A 61 K 31/43
C 07 D 205/08
C 07 D 498/04

TECHNICAL FIELDS SEARCHED (Int.Cl. 3)

C 07 D 499/00
A 61 K 31/00
C 07 D 205/00
C 07 D 498/00
C 07 D 487/00

CATEGORY OF CITED DOCUMENTS

X: particularly relevant
A: technological background
O: non-written disclosure
P: intermediate document
T: theory or principle underlying the invention
E: conflicting application
D: document cited in the application
L: citation for other reasons

&: member of the same patent family, corresponding document

X The present search report has been drawn up for all claims

| Place of search | Date of completion of the search | Examiner |
|---|---|---|
| VIENNA | 03-12-1979 | JANISCH |